# NEW EUROPEAN PATENT SPECIFICATION

(11) **EP 0 296 275 B2**
(45) Date of publication and mention of the opposition decision: **09.10.1996**
(45) Mention of the grant of the patent: 24.03.1993
(21) Application number: 87110086.3
(22) Date of filing: 13.07.1987
(51) Int. Cl.: C07C 53/128, C07C 51/14

(54) **Carboxylic acid mixture and process for producing the same**
Carbonsäuregemisch und Verfahren zu seiner Herstellung
Mélange d'acides carboxyliques et procédé de préparation

(30) Priority: 23.06.1987 JP 156089/87; 30.06.1987 JP 165057/87
(43) Date of publication of application: 28.12.1988
(73) Proprietor: IDEMITSU PETROCHEMICAL CO. LTD., Tokyo 100 (JP)
(72) Inventor: Kawasaki, Hiroshi, Tokuyama-shi Yamaguchi-ken (JP); Miwa, Hideaki, Tokyo (JP); Ichihara, Osamu, Tokuyama-shi Yamaguchi-ken (JP)
(74) Representative: TER MEER - MÜLLER - STEINMEISTER & PARTNER

(56) References cited:
- GB-A- 1 016 087
- GB-A- 1 182 519
- GB-A- 1 218 154
- JP-A- 197 894
- US-A- 2 876 241
- US-A- 3 910 963
- US-A- 4 002 577
- CHEMICAL ABSTRACTS, vol. 89, no. 13, September 25, 1978, page 789, abstract no. 108019q, Columbus, Ohio, US; Y. SOUMA et al.: "Carbonylation of olefin and alcohol catalyzed by copper (I) carbonyl in hydrogen fluoride"
- PATENT ABSTRACTS OF JAPAN, vol. 12, no. 5, (C-467)(2852), January 8, 1988 & JP-A-62164645
- PATENT ABSTRACTS OF JAPAN, vol. 12, no. 47 (C-475)(2894), February 12, 1988 & JP-A-192338
- Journal of the Japanese Chemical Society, 1972, pp. 1472-1480, M. YOSHIHISA et al, "Synthese verzweigter aliphatischer Säuren aus Wasser, Kohlenmonoxid und Isobutylen" and translation into German thereof
- M. MUTSUYA et al, Koka, 73, p. 2147, 1970
- J. FALBE, "New syntheses with carbon monoxide", Springer Verlag Berlin, Heidelberg, New York, 1980, pp. 384-386, 395-396, 408-413
- C, H. ROCHESTER, Acidity Functions, 1970, pp. 23, 26, 28, 50-53
- J. FALBE, "Carbon monoxide in organic synthesis", 1970, pp. 144-145

## Description

This invention relates to a process for the production of a carboxylic acid mixture which contains at least 55% by weight of an isomer mixture of carboxylic acids selected from the group comprising isomer mixtures of tertiary carboxylic acids having 9 carbon atoms and isomer mixtures of tertiary carboxylic acids having 13 carbon atoms, which carboxylic acids are difficult to esterify, which mixture has a lower content of impurities, such as sulfur compounds.

Esters of tertiary carboxylic acids are widely used as ingredients for coating compositions, solvents, etc..

Esters of tertiary carboxylic acids for these uses are required to be of high level in hydrolysis resistance because they are exposed to severe conditions for a long time.

It has been known that a carboxylic acid mixture mainly composed of the tertiary carboxylic acids used for production of tertiary acid esters for the above uses is prepared by the so-called Koch's reaction.

This Koch's reaction comprises reacting an olefin or an alcohol, carbon monoxide and water in the presence of an acid catalyst containing hydrochloric acid, sulfuric acid, phosphoric acid or boron trifluoride to produce a carboxylic acid having one more carbon atom than the starting olefin or alcohol.

Processes for production of tertiary carboxylic acids include the following various processes.

For example, Japanese Patent Examined Publication No. 35048/73 proposes a process for the production of a branched carboxylic acid which is characterized in that prior to destillation of a crude fatty acid obtained by removing sulfuric acid catalyst from a Koch's reaction product obtained by reaction of an olefin with carbon monoxide and water in the presence of sulfuric acid catalyst, said crude fatty acid is heated in the liquid phase at 100-270° C together with water or water containing a small amount of an organic acid salt or an inorganic salt.

This patent publication discloses, as examples of sulfuric acid catalysts, an aqueous sulfuric acid solution of more than about 80% by weight in concentration or concentrated suifuric acid or mixtures of these sulfuric acids with at least one of boron trifluoride, hydrogen fluoride and phosphoric acid. However, as shown in Example 1, the process is carried out under high pressure condition of as high as 69 bar (70 kg/cm²) in carbon monoxide pressure. Besides, the products are crude fatty acid mixtures containing 41.8% by weight of trichloroethylene, 42.3% by weight of branched C9 carboxylic acids and only 6.2% by weight of C13 carboxylic acids and as a result of purification of the crude fatty acid mixture, there was merely obtained a C9 carboxylic acid containing 49.4% by weight of 2,2,4,4-tetramethylpentanoic acid and 44.45% by weight of a mixture of 2,3,3-trimethyl-2-ethylbutanoic acid, 2,2-diisopropylpropanoic acid, 2,2,3,4-tetramethylpentanoic acid and 2,2,3,3-tetramethylpentanoic acid. Thus, the carboxylic acid mixture, which gives esters difficult to hydrolyze, was not obtained at the rate of more than 50%. A similar result was obtained in Example 2.

On the other hand, Japanese Patent Examined Publication No. 3362/55 discloses a process for the manufacture of a carboxylic acid from an olefin and carbon monoxide in the presence of at least 90% sulfuric acid or anhydrous hydrogen fluoride alone or mixed with boron fluoride as a catalyst, characterized in that the reaction is first carried out in the liquid phase without adding water and then the reaction product is taken in water and treated at room temperature.

This publication discloses an example (Example 4) for the production of carboxylic acids mainly composed of trimethylacetic acid by introducing isobutene into an autoclave containing carbon monoxide kept at 49 bar (50 atm) in the presence of 97% sulfuric acid and carrying out the reaction at 6-10° C for 4.5 hours. In other examples, there were also produced carboxylic acids which provide esters which are easily hydrolyzed. Thus, this publication disc!oses a process for production of easily hydroiyzed carboxylic acids and does not disclose those which provide esters which cannot be easily hydrolyzed as in this invention.

Further, Japanese Patent unexamined Publication (Laid-Open) No. 48616/74 discloses a process for the production of tertiary carboxylic acids which comprises subjecting alcohols or olefins of at least 4 carbon atoms in coexistence of copper, silver and gold alone or mixed with a monovalent compound (excluding halides, cyanides and nitrate) using at least 80% sulfuric acid, hydrogen fluoride, fluorosulfuric acid, phosphoric acid or boron fluoride complex as a solvent under a carbon monoxide partial pressure of 0.1 bar (atm.) or higher and at -10° C∼70° C.

However, Example 2 of this publication mentions the production of a mixture (3:2) of 2,2-dimethyl-nonanoic acid and 2-methyl-2-ethyloctanoic acid from heptene-1 using 100% sulfuric acid as a solvent and cuprous oxide and aurous oxide as catalyst; Example 4 shows the production of pivalic acid from butene-1 in the yield of about 40% based on the olefin used using 88% sulfuric acid as a solvent and cuprous oxide and silver oxide as catalyst;and Example 6 shows the production of 3:1 mixture of 2,2-dimethylheptanoic acid and 2-methyl-2-ethyl-hexanoic acid from 2-ethylhexene-1 using phosphoric acid as a solvent and a complex of boron fluoride and acetic acid and cuprous oxide as catalyst. All of these examples merely show the production of tertiary carboxylic acids which provide esters easily hydrolyzable.

Norihiko Yoneda et al, "Chemistry Letters", 1984, p 607-610 discloses the carbonylation reaction of diisobutylene with carbon monoxide in BF₃-H₂O using Cu(l) carbonyl as a catalyst.

According to the process for production of carboxylic acids from diisobuthylene shown in the above literature, carboxylic acids are produced in a yield of 77% in case of a reaction without solvent and the composition of the carboxylic acids is C5 carboxylic acid 32%, C6-8 carboxylic acid 13%, C9 carboxylic acid 36% and other carboxylic acids 19% and the carboxylic acids are produced in a yield of 80-82% in case of the reaction in a solvent such as cyclohexane or chlorobenzene and the composition of the carboxylic acids is C5 carboxylic acid 41-44%, C6-8 carboxylic acid 11-14%, C9 carboxylic acid 35-36% and other carboxylic acids 9-10%. Thus, the process disclosed in this literature is low in yield of carboxylic acids and besides produces only the products mainly composed of carboxylic acids which provide easily hydrolyzed esters.

Yoshie Souma et al, "Oil Chemistry", Vol.30, No.5, 1981, p 265 and Yoshie Souma. "Organic Synthetic Chemistry", Vol.41, p 561-569, 1983 also disclose the synthesis of carboxylic acids from oiefins with metallic carbonyl catalysts, but the yield of the carboxylic acids is low and can afford only the products mainly composed of such carboxylic acids as providing esters which are easily hydrolyzed.

Japanese Patent examined Publication No. 16897/73 discloses a purification which is characterized by contacting an oxidizing agent with a crude fatty acid obtained by removing a sulfuric acid catalyst from a so-called Koch's -eaction product obtained by reacting an olefin with carbon monoxide and water in the presence of said sulfuric acid catalyst prior to distillation of the crude fatty acid.

GB-A-1 016 087 discloses a process for producing organic carboxylic acids using sulfuric acid as a catalyst.

US-A-4 002 577 describes a catalyst composition useful for the treatment of olefins or alcohols with carbon monoxide in the preparation of carboxylic acids consisting essentially of a solution of hydrated boron trifluoride and a minor amount of copper carbonyl. This catalyst optionally may contain a mineral acid, such as sulfuric acid, phosphoric acid or hydrogen fluoride.

US-A-3 910 963 covers a method for the manufacture of carboxylic acids or derivatives thereof by means of the Koch reaction using strong acids, such as sulfuric acid, hydrogen fluoride, fluoral sulfonic acid or a complex of phosphoric acid and boron fluoride as a catalyst.

JP 197 894184 discloses a method for the preparation of tertiary carboxylic acids which is characterized in that the complex of tertiary carboxylic acid and sulphuric acid, obtained by subjecting the product of reaction between olefin and carbon monoxide catalyst by the presence of sulphuric acid cuprous oxide, to an extraction treatment, is subjected to a water wash so as to recover the sulphuric acid for recycling to the reaction system.

### SUMMARY OF THE INVENTION

An object of this invention is to provide a process for the production of a carboxylic acid mixture which provides esters which are not easily hydrolyzed.

These objects are attained by the process according to claim 1 and 2. Sub-claims 3 to 6 comprise preferred embodiments of said process.

According to a preferred embodiment of this process the catalyst additionally contains a metal compound, such as a metal oxide, preferably cuprous oxide. According to a further preferred embodiment the content of said metal compound is 0.2-2% by weight of the mixture of sulfuric acid and phosphoric acid.

### DESCRIPTION OF THE INVENTION

Said carboxylic acid mixture contains at least 55% by weight of an isomer mixture of carboxylic acids which are difficult to esterify.

The expression "carboxylic acid difficult to esterify" used herein can be defined as follows:

A reaction product obtained when a carboxylic acid is reacted with methanol in an amount of 4 times the weight of the carboxylic acid with the assistance of zinc acetate used as a catalyst in an amount of 5.4% by weight of the carboxylic acid at a reaction temperature of 200°C for a reaction time of 5 hours is analyzed by gas chromatography under the following analytical conditions and when the conversion rate of the starting carboxylic acid to carboxylic acid ester is 40% or less, it is defined that the carboxylic acid is difficult to esterify.

### Analytical conditions

| | |
|---|---|
| Gas chromatography device: | Type GC-9A (Shimazu Seisakusho Co.) |
| Column packing material: | FON (Trade name for product of Shimazu Seisakusho Co.) |
| Column diameter: | 3 φ x 2m |
| Column temperature: | Elevated from 100°C to 250°C. |
| Temperature elevation rate: | 10°C/min |
| Detector: | FID |
| Injection: | 250°C |

It can be presumed that since the carboxylic acid mixture containing at least 55% by weight of a mixture of isomers of carboxylic acids which are difficult to esterify contains, as the main component, tertiary carboxylic acids the carboxyl group of which is surrounded by bulky groups, the carboxylic acids are not easily esterified under said esterification conditions due to the steric hindrance. The tertiary carboxylic acids having steric hindrance provide such a characteristic that when they are esterified under esterification conditions severer than said esterification conditions, the esters do not easily undergo hydrolysis. That is, esters of tertiary carboxylic acids having steric hindrance have hydrolysis resistance.

Here, the hydrolysis resistance can ben defined as follows:

A methyl ester of a carboxylic acid is added to a 0.1 N sodium hydroxide solution (in mixture of water: ethylene glycol monomethyl ether ≃1:10) and the mixture is left to stand in a thermostatic chamber at 80° C for 50 hours to carry out alkali hydrolysis. The resulting reaction product is analyzed by gas chromatography under the following analytic conditions and when conversion rate of the starting carboxylic acid ester to carboxylic acid is 40% or less, this carboxylic acid is defined to be difficult to hydrolyze.

### Analytic condtions

| | |
|---|---|
| Gas chromatography device: | Type GC-9A (Shimazu Seisakusho Co.) |
| Column packing material: | FON (Trade name for product of Shimazu Seisakusho Co.) |
| Column diameter: | 3φx 2m |
| Column temperature: | Elevated from 100°C to 250°C. |
| Temperature elevation rate: | 10°C/min |
| Detector: | FID |
| Injection: | 250°C |

Therefore, the carboxylic acid mixture produced according to this invention is useful as chemically stable coating compositions and solvents when the mixture is esterified.

As the isomer mixtures of carboxylic acids difficult to esterify, mention may be made of isomer mixtures of tertiary carboxylic acids of 9 carbon atoms, those of 13 carbon atoms, and those of 9 carbon atoms and 13 carbon atoms.

The isomer mixtures of tertiary carboxylic acids of 9 carbon atoms include, for example, 2-ethyl-2,3,3-trimethylbutanoic acid, 2-isopropyl-2,3-dimethylbutanoic acid, 2,2,3,3-tetramethylpentanoic acid, 2,2,3,4-tetramethylpentanoic acid, etc.

The isomer mixtures of tertiary carboxylic acids of 13 carbon atoms include, for example, 2.2,4,4,6,6 -hexamethylheptanoic acid, 2-ethyl-2,3,3,5,5 -pentamethylhexanoic acid, 2,4,4-trimethyl-2-t-pentylpentanoic acid, 2-isopentyl-2,4,4-trimethylpentanoic acid, 2-isopropyl-2,3,5,5-tetramethylpentanoic acid, etc.

The isomer compounds of tertiary carboxylic acids having 9 carbon atoms and having 13 carbon atoms include the above enumerated tertiary carboxylic acids.

The carboxylic acid mixture produced by the process of this invention may contain other components such as polymerized oils, carboxylic acids having 5-8 or 14 carbon atoms or carboxylic acids which provide esters which are easily hydrolyzed as far as it contains at least 55% by weight of isomer mixture of carboxylic acids difficult to esterify.

Furthermore, carboxylic acid mixtures which are increased in contents of tertiary carboxylic acids of 9 carbon atoms, those of 13 carbon atoms, or those of 9 carbon atoms and 13 carbon atoms by subjecting the carboxylic acid mixtures of this invention which contain polymerized oils or carboxylic acids of 5-8 carbon atoms or 14 carbon atoms to purification operation may also be inc!uded in the carboxylic acid mixtures produced according to this invention to as far as they contain at least 55% by weight of an isomer mixture of carboxylic acids difficult to esterify.

Olefins used as starting materials in the process of this invention are selected from the group comprising diisobutylene and triisobutylene.

For diisobutylene, preferred are diisobutylene mixtures containing totally 80% or more of 2,4,4-trimethylpentene-1 and 2,4,4-trimethylpentene-2 and totally 5-15% of 2,3,4-trimethylpentene-1 and 2,3,4-trimethylpentene-2 and for triisobutylene, preferred are triisobutylene mixtures containing 80% or more of branched olefins having 12 carbon atoms such as 2,4,4,6,6-pentamethyl.1-hexene and 2,4,4,6,6-pentamethyl-2-hexene.

Carbon monoxide used in the process of this invention is most preferably pure carbon monoxide, but carbon monoxide-containing gases obtained from water gas, generator gas, coke oven gas etc. may also be used.

Water used in the process of this invention is most preferably pure water, but distilled water, deionized water, etc. may also be employed. The catalyst used in the process of this invention are selected from the group consisting of a catalyst comprising sulfuric acid and phosphoric acid, and a catalyst comprising sulfuric acid, phosphoric acid and a metal compound,.

Said metal compounds include, for example, metal oxides such as cuprous oxide, silver oxide, gold oxide, etc. and preferred is cuprous oxide. These metal oxides may be used as mixtures with metallic copper or divalent copper compounds.

In case of using the catalyst comprising sulfuric acid and phosphoric acid, or the catalyst comprising sulfuric acid, phosphoric acid and a metal compound, it is necessary that the acid strength of the catalyst is within the range of -9.2∼-6, preferably -9.0∼6.5.

The acid strength used here means Hammett's acidity function Ho.

When the acid strength of the catalysts used is higher than -6, the carbonylation reaction is difficult to take place and the polymerization or isomerization of olefins tends to occur. When it is lower than -9.2, the carbonylation of isobutylene oligomer with keeping its skeleton is apt to occur in case of using isobutylene oligomer as the olefin and furthermore, the cleavage of isobutylene monomer vigorously occurs to increase the tendency of production of much pivalic acid (C5 acids).

When, for example, a catalyst comprising sulfuric acid and phosphoric acid of an acid strength within said range is used, the concentration of sulfuric acid in the catalyst is usually 30-90% by weight, preferably 40-80% by weight.

In this mixture of sulfuric acid and phosphoric acid there may be present water which is contained in sulfuric acid and phosphoric acid or added separately. Content of this water is usually not more than 25% by weight.

The concentration of sulfuric acid, and of phosphoric acid that provides the acid strength within the range mentioned above can be optionally determined by experiments.

There are no limitations in the method of preparation of catalysts. For example, previously sulfuric acid, and phosphoric acid are mixed by ordinary mixing operation to prepare a catalyst solution containing sulfuric acid and phosphoric acid and this may be used as it is or after dilution.

Further, sulfuric acid, phosphoric acid and a metal compound are previously mixed and this catalyst solution containing the metal compound may be used as above.

Preferably, the starting materials are added to the catalyst components which have been charged in a reaction vessel.

When a metal compound, especially a metal oxide, especially cuprous oxide is used as a component of the catalyst, the amount of this metal compound is normally 0.1-4% by weight, preferably 0.2-2% by weight of sulfuric acid and phosphoric acid.

If the amount of the metal compound is less than 0.1% by weight, there is the tendency of an increase in the production of olefin-polymerized oil and even if it is more than 4% by weight, the reaction result is not improved so much and rather the production of water may cause an increase of the acid strength of the catalysts and a decrease of carbonylation activity.

In the process of this invention, reaction pressure(gage pressure) is 2-39 bar (2-40 kg/cm²), preferably 4-40 bar (4-20 kg/cm²).

When diisobutylene is used as a starting material and reaction pressure is more than 39 bar (40 kg/cm²), carboxylic acids which provide esters inferior in hydrolysis resistance, e.g., 2,2,4,4-tetramethylpentanoic acid are produced in a large amount. This is considered because under relatively higher pressure exceeding 39 bar (40 kg/cm²), isobutylene oligomer is converted, with maintaining its skeleton, to 2,2,4,4-tetramethylpentanoic acid while under relatively low pressure of 2-39 bar (2-40 kg/cm²), the carbon skeleton of the isobutylene oligomer is converted to a carboxylic acid chemically more stable, in other words, more inert than 2,2,4,4-tetramethylpentanoic acid due to repeated isomerization. On the other hand, when reaction pressure is lower than 2 bar (2 kg/cm²), polymerization of only olefin takes place and the reaction may not proceed sufficiently.

The reaction temperature may be selected from the range of -10°C∼-80°C and generally is 0∼50°C. When it is lower than 0°C, reaction speed may decrease. When higher than 50°C, there may occur increase of sulfuric acid sludge, sulfates, etc.

The reaction time is ordinarily such that is sufficient to secure 45-120 minutes as feeding time of olefin or alcohol and 30-120 minutes of stirring time.

The reaction of the olefin or the alcohol with carbon monoxide and water in the presence of said catalyst may be effected by any of batch process, semi-batch process and continuous process.

After the completion of reaction, a carboxylic acid mixture further increased in content of carboxylic acid which is difficult to esterify can be obtained by subjecting the resulting reaction product to purification operations such as extraction with organic solvent such as n-hexane, washing with water and removal of the extraction solvent.

According to this invention, the object of this invention producing on isomer mixture of carboxylic acids which are esterified with difficulty is attained by using a catalyst comprising of sulfuric acid, phosphoric acid and, if necessary, metal compound is used. The reaction product solution obtained contains less sulfur compounds which originate from sulfuric acid which is one of catalyst components and hence, less sulfur compounds incorporate into carboxylic acid, resulting in carboxylic acid mixtures free from coloration and malodor.

Why the use of said catalysts can restrain the production of sulfur compounds is not clear.

However, even without theoretical elucidation for the mechanism of restraint of the production of sulfur compounds, the process of this invention can be considered to be industrailly advantageous process for production of carboxylic acids because according to the process of this invention, it is possible to produce carboxylic acids free from malodor and coloration in high yields with restraining formation of sulfur compounds.

In the process of this invention, it is possible to recover the catalyst solution from said extraction solvent and reuse it by recycling this recovered catalyst solution to the reaction system. In this respect, this invention also has an advantage. When a metal compound is used as one of catalyst components, the recovery of catalyst solution is preferably carried out in an atmosphere where the metal compound cannot be oxidized. If the recovery is carried out in an oxidizing atmosphere, the metal compound is oxidized to cause a reduction of the catalyst activity and when the recovered catalyst is reused, sometimes the yield of carboxylic acid decreases.

The carboxylic acid mixtures prepared according to this invention have the following excellent characteristics.
(1) They contain tertiary carboxylic acids having steric hindrance as main component and so are stable carboxylic acid mixtures which are not easily esterified.
(2) Therefore, they are carboxylic acid mixtures which are not easily esterified due to the steric hindrance and which provide esters excellent in hydrolysis resistance.

The process of this invention has the following advantages.
(1) It is possible to selectively produce carboxylic acid mixture containing at least 55% by weight of carboxylic acids which provide esters having hydrolysis resistance.
(2) It is industrially advantageous since the reaction can be carried out under a pressure of 2-39 bar (2-40 kg/cm²).
(3) It is economical process because the catalyst used in production can be recovered and reused. This invention is further illustrated by the following examples and comparative examples.

### Example 1

In a one-liter magnetic stirring type stainless steel autoclave were charged 270 g of a strong acid mixture composed of 64% by weight of sulfuric acid, 29% by weight of phosphoric acid and 7% by weight of water and 2.3 g of cuprous oxide and the inside of the autoclave was sufficiently purged with carbon monoxide gas, followed by stirring at 1000 rpm for 3 hours with keeping a temperature of 25°C and a carbon monoxide pressure of 15 bar (15 kg/cm²) to completely dissolve cuprous oxide.

Subsequently, with keeping said temperature and pressure, 33.6 g of diisobutylene was fed to the autoclave over a period of 90 minutes. The composition of diisobutylene used was 2,4,4-trimethyl-1-pentene: 70.9% by weight, 2,4,4-trimethyl-2-pentene: 22.1% by weight and other olefins having 8 carbon atoms: 7.0% by weight. Carbon monoxide consumed by the reaction was replenished so as to keep the carbon monoxide pressure at 15 bar (15 kg/cm2). After feeding of diisobutylene was discontinued, stirring was continued for further 1 hour. An acid strength of the catalyst varied from -8.1 to -7.5 before and after this reaction.

After completion of the reaction, the resulting reaction mixture was diluted to 3 times with water and repeatedly extracted 3 times with 200 ml of n-hexane. n-Hexane was distilled out to obtain 47.1 g of a crude carboxylic acid mixture of the composition shown in Table 1.

**Table 1**

| COMPONENTS | COMPOSITION (% by weight) |
|---|---|
| Polymerized oil | 0.5 |
| Pivalic acid | 8.2 |
| C6 acids | 1.9 |
| C7,8 acids | 2.6 |
| C9 acids | 59.4 |
| C10-13 acids | 26.8 |
| C14 acids | 0.6 |
| Note: "C6", "C7,8", "C9", "C10-13", "C14" in the above table mean 6 carbon atoms, 7 or 8 carbon actoms, 9 carbon atoms, 10-13 carbon atoms and 14 carbon atoms, respectively. | |

This crude carboxylic acid mixture was subjected to distillation under reduced pressure to obtain 28.0 g of a fraction at 85-101° C 1.3 mbar (1 mmHg).

This fraction was analyzed by gas chromatography to find that this was a carboxylic acid mixture composed of 31% by weight of a mixture of 2,2,4,4-tetramethylpentanoic acid and other carboxylic acids (referred to as "carboxylic acid(A)") and 69% by weight of a mixture composed of 2-ethyl-2,3,3-trimethylbutanoic acid, 2-isopropyl-2,3-dimethylbutanoic acid, 2,2,3,3-tetramethylpentanoic acid, 2,2,3,4-tetramethylpentanoic acid and otner tertiary carboxylic acids (referred to as"carboxyic acid(B)").

The analytic conditions were the same as those given in definition of difficulty in esterification hereinbefore.

Then, 50.0 g of this carboxylic acid mixture, 200 g of methanol and 2.7 g of zinc acetate were charged in a one-liter autoclave, followed by purging with nitrogen and then reaction was effected at 200° C over a period of 5 hours. The reaction product was analyzed by gas chromatography to find that 15% of carboxylic acid (B) was esterified and 80% of carboxylic acid (A) was esterified.

Therefore, according to the definition of difficulty in esterification, the carboxylic acid mixture obtained in this example was a carboxylic acid mixture containing at least 55% of isomer mixture of tertiary carboxylic acids which were difficult to esterify.

### Example 2

A crude carboxylic acid mixture having the composition shown in Table 2 was obtained in the same manner as in Example 1 except that 52.4 g of triisobutylene having the composition of C11-olefin 0.8% by weight, C12-olefin 94.3% by weight and C13-olefin 4.9% by weight was used in place of 33.6 g of diisobutylene and reaction temperature was kept at 5°C. The acid strength of the catalyst varied from -8.1 to -7,6 before and after this reaction.

**Table 2**

| COMPONENTS | COMPOSITION (% by weight) |
|---|---|
| Polymerized oil | 1.1 |
| Pivalic acid | 7.6 |
| C6 acids | 2.5 |
| C7,8 acids | 3.2 |
| C9 acids | 17.8 |
| C10-13 acids | 66.5 |
| C14 acids | 1.3 |

This crude carboxylic acid mixture was subjected to distillation under reduced pressure to obtain 46.6 g of a fraction at 119-127°C 1.3 mbar (1 mmHg).

This fraction was analyzed by gas chromatography to find that this was a carboxylic acid mixture (referred to as "carboxylic acid (B')) composed of 2,2,4,4,6,6-hexamethylbutanoic acid, 2-ethyl-2,3,3,5,5-penthylpentanoic acid, 2,4,4-trimethyl-2-t-pentylpentanoic acid, 2-isopentyl-2,4,4-trimethylpentanoic acid, 2-isopropyl-2,3,5,5-tetramethylhexanoic acid and other tertiary carboxylic acids.

The analytic conditions by gas chromatography were the same as those used for the definition of difficulty in esterification given hereinbefore.

Then, esterification reaction was effected in the same manner as in Example 1 except that said carboxylic acid mixture (carboxylic acid (B')) was used. The reaction product was analyzed by gas chromatography to find that 24% of carboxylic acid(B') was esterified.

Therefore, according to the definition of difficulty in esterification given hereinbefore, this carboxylic acid (B') obtained in this Example 2 was a carboxylic acid mixture containing at least 55% by weight of isomer mixture of tertiary carboxylic acids difficult to esterify.

### Comparative Example 1

Esterification reaction is carried out in the same manner as in Example 1 except that pivalic acid was used.

According to analysis of the reaction product by gas chromatography (Analytical conditions were same as given in definition of difficulty in esterification hereinbefore except that column was heated at 55°C for 5 minutes and then the temperature was elevated to 250°C.), 94% of pivalic acid was converted to methyl pivalate.

### Example 3

150 ml of a catalyst comprising an aqueous solution containing 64% by weight of sulfuric acid and 29% by weight of phosphoric acid was charged in a reaction vessel, which was fully purged with carbon monoxide, followed by absorption of carbon monoxide to saturation.

Then, 0.3 mol of diisobutylene was fed to the reaction vessel with keeping a reaction temperature of 25°C and a reaction pressure of 5 bar (5 kg/cm²) to react diisobutylene with carbon monoxide. The carbon monoxide consumed by the reaction was replenished so as to maintain the given pressure.

After lapse of a reaction time of 1.5 hour, the feeding of diisobutylene was discontinued and stirring was effected for further 1 hour. Then, an equal amount of water was added.

Thus obtained reaction mixture was diluted to 3 times with water and then extracted with 200 ml of n-hexane repeatedly 3 times.

Then, n-hexane was distilled out to obtain a carboxylic acid of the composition as shown in Table 3.

The results are shown in Table 3.

The composition of the reaction product was measured by NMR spectrum.

### Examples 4 and 5

Example 3 was repeated except that the reaction pressure as shown in Table 3 was employed.

The results are shown in Table 3.

### Comparative Examples 2 and 3

Example 3 was repeated except that a catalyst comprising an aqueous solution containing 85% by weight of sulfuric acid and the reaction pressure as shown in Table 3 was employed. The results are shown in Table 3.

As is clear from Table 3, in both of these comparative examples, the amount of 2,2,4,4-tetramethylpentanoic acid produced which was insufficient in hydrolysis resistance when esterified was greater than in Example 3.

### Comparative Examples 4 and 5

Example 3 was repeated except that reaction pressure as shown in Table 3 was employed. The results are shown in Table 3.

As is clear from Table 3, in both of these comparative examples, amount of 2,2,4,4-tetramethylpentanoic acid which was insufficient in hydrolysis resistance when esterified was greater than in Example 3.

### Reference Example 1

In a one-liter stainless steel autoclave were charged 200 ml of methanol and 50.0 g of a mixture (carboxylic acid (A): carboxylic acid (B)=53 mol% : 47 mol%) of 2,2,4,4-tetramethylpentanoic acid (referred to as "carboxylic acid(A)" in this example and Table 3) synthesized in Comparative Example 5 using a catalyst (acid strength: -8.1--7,5) comprising a mixed solution of sulfuric acid and phosphoric acid and a mixture of 2-ethyl-2,3,3-trimethylbutanoic acid, 2-isopropyl-2,3-dimethylbutanoic acid, 2,2,3,3-tetramethylpentanoic acid, 2,2,3,4-tetramethylpentanoic acid and other carboxylic acids (referred to as "carboxylic acid- (B) in this example and Table 3) and these were stirred at 225°C for 12 hours to carry out methyl esterification reaction.

The resulting product was analyzed by gas chromatography to find that the conversion rate to the methyl ester was 56%. Further, unreacted acids were analyzed by gas chromatography to find that the ratio of carboxylic acid (A) and carboxylic acid (B) changed to 17 mol% : 83mol%.

From the above analytical results and the definition of easiness in esterification, it was clarified that carboxylic acid (B) was markedly lower in esterification reactivity than carboxylic acid (A).

### Reference Example 2

6.7 g of a carboxylic acid mixture (carboxylic acid (A) : carboxylic acid (B) = 53 mol% : 47 mol%) prepared in the same manner as in Comparative Example 5 using a catalyst (acid strength : -8.1--7.5) comprising a mixed solution of sulfuric acid and phosphoric acid and 50 ml of hexamethylphosphoric triamide were charged in a 100 ml three necked flask, followed by adding 25% aqueous solution containing 2.43 g of sodium hydroxide and stirring at room temperature for 30 minutes. Further, 10 g of methyl iodide was added thereto. The solution immediately became cloudy to precipitate sodium iodide.

The reaction mixture was put in 50 ml of water and extracted with ether. The extraction solution was washed with water and then distilled to obtain 7.2 g of methyl ester of carboxylic acid (A) : ester of carboxylic acid (B) = 53 mol% : 47 mol%.

Then, 1.2 ml of this methyl ester mixture was taken in a 500 ml flask and thereto was added 400 ml of 0.1 N NaOH solution (mixed liquid of water: ethylene glycol monomethyl ether = 1 : 10) and then this was left to stand in a thermostatic chamber of 80°C for 50 hours to carry out alkali hydrolysis.

Thereafter, the reaction mixture was subjected to analysis by gas chromatography to find that the ratio of ester of carboxylic acid (A) and ester of carboxylic acid (B) changed to 27 mol% : 78 mol%.

From this result and the definition of hydrolysis resistance given hereinbefore, it was clear that the ester of carboxylic acid (A) was more susceptible to hydrolysis than the ester of carboxylic acid (B).

### Examples 6-9 and Comparative Examples 6-9

Aqueous sulfuric acid solution and aqueous phosphoric acid solution of the concentrations as shown in Table 4 (phosphoric acid was not used in Comparative Examples) and cuprous oxide in the amount as shown in Table 4 and catalyst in the amount as shown in Table 4 were charged in a reaction vessel and then the reaction vessel was fully purged with carbon monoxide and, followed by absorption of carbon monoxide to saturation with stirring. Then, diisobutylene was fed to the reaction vessel with maintaining a carbon monoxide pressure of 15 bar (15 kg/cm²) and reacted with carbon monoxide under the reaction conditions as shown in Table 4. Fresh carbon monoxide was replenished for carbon monoxide consumed by the reaction. After the lapse of the reaction time of 1.5 hour, feeding of diisobutylene was discontinued and stirring was effected for further 1 hour. Then, an equal amount of water was added.

The reaction mixtures obtained in Examples 6 and 7 and Comparative Examples 6 and 7 , as they were, without dilution with water and those obtained in Examples 8 and 9 and Comparative Examples 8 and 9 diluted to 3 times with water were extracted with 200 ml of n-hexane repeatedly 3 times.

Some sulfuric acid incorporated into the extracts was removed by washing with water and n-hexane was removed by distillation to obtain carboxylic acid mixtures of the compositions as shown in Table 4.

The results are shown in Table 4.

The content of sulfur in the resulting carboxylic acid mixtures was measured by trace sulfur analyzer and the content of phosphorus was determined by incinerating the carboxylic acid mixtures in an electric furnace, collecting the incinerated product, adding an indicator thereto and measuring the absorbance for coloration.

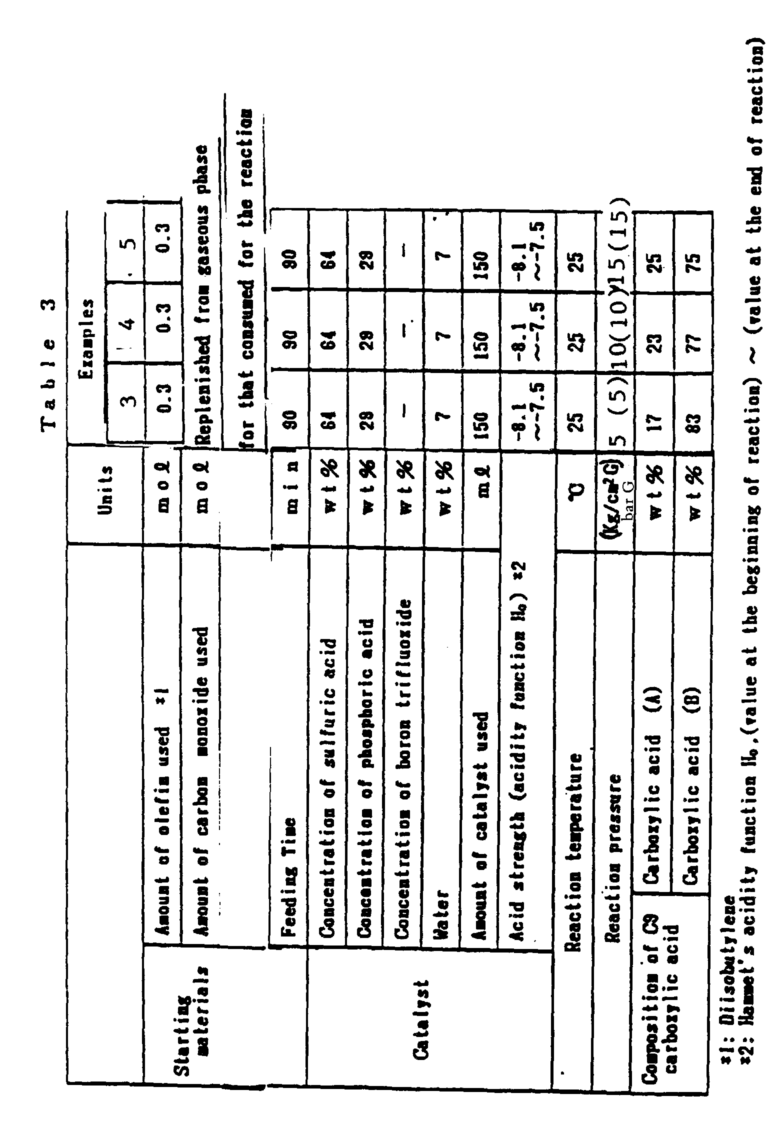

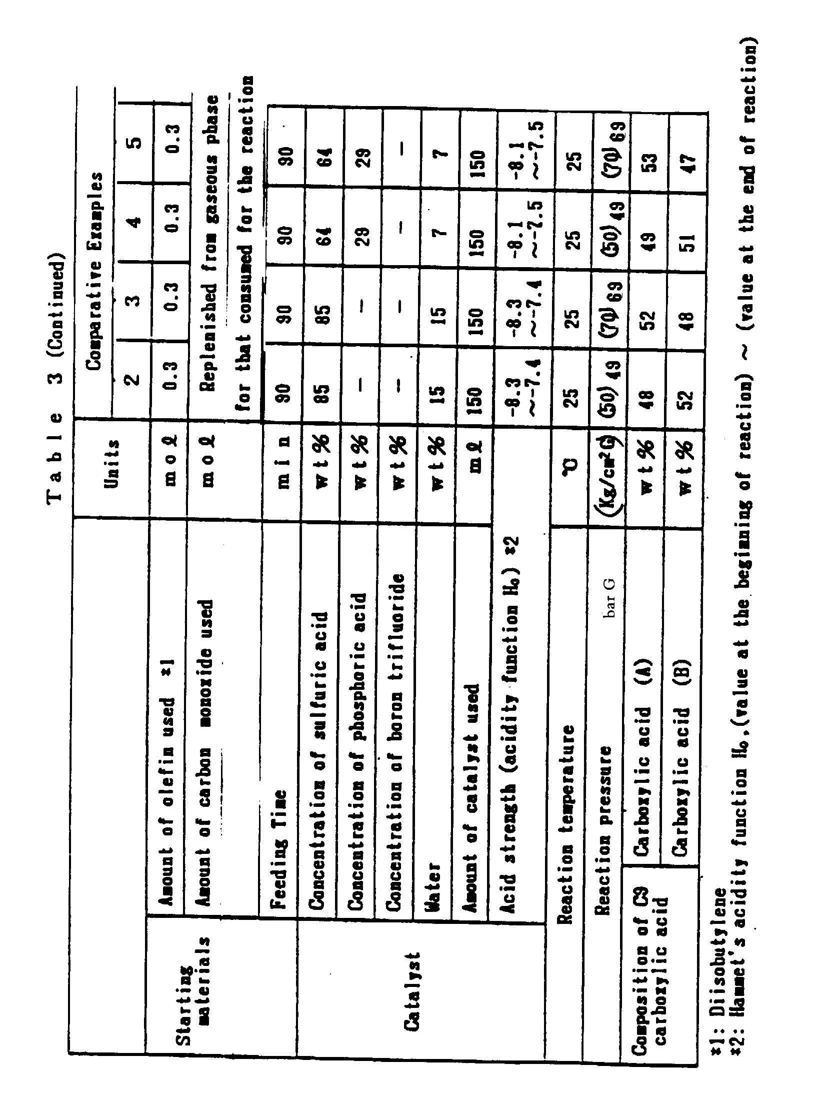

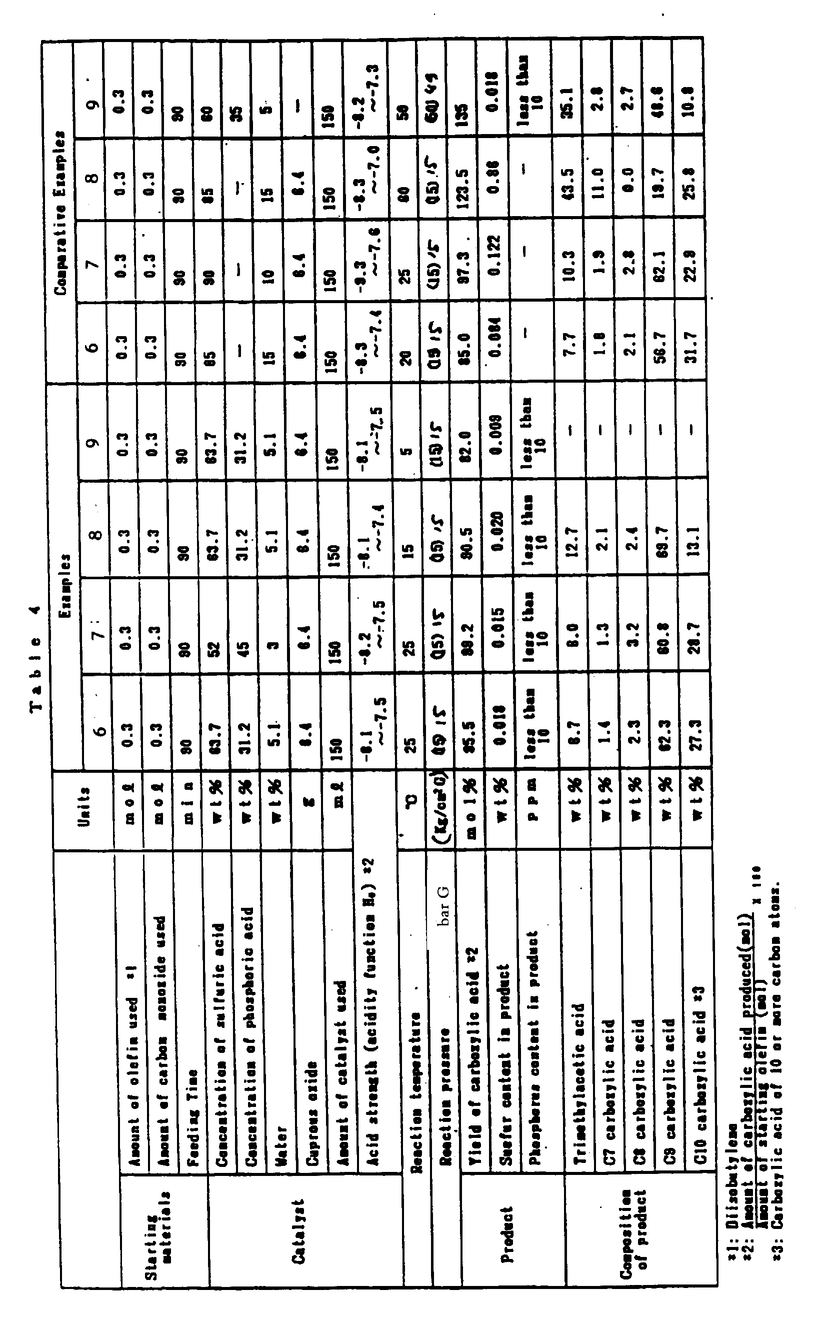

## Claims

1. A process for the production of a carboxylic acid mixture which contains at least 55 % by weight of an isomer mixture of tertiary carboxylic acids having 13 carbons atoms, said carboxylic acids being so difficult to be esterified that the reaction product obtained by reacting said carboxylic acids with methanol in an amount of 4 times the weight of the carboxylic acids in the presence of zinc acetate as a catalyst in an amount of 5.4 % by weight of the carboxylic acids at a reaction temperature of 200°C and a reaction time of 5 hours has according to gas chromatography a conversion rate of the starting carboxylic acids to the carboxylic acid esters of 40 % or less, said process comprises reacting a triisobutylene, carbon monoxide and water in the presence of a catalyst consisting of sulfuric acid and phosphoric acid under a pressure of 2-39 bar (2-40 kg/cm²), wherein the concentration of said sulfuric acid in the catalyst is 30-90 % by weight and the concentration of said phosphoric acid in the catalyst is 5-70 % by weight so that an acid strength by Hammett's acidity function Ho of the catalyst is within the range of from -9.2 to -6.

2. A process for the production of a carboxylic acid mixture which contains at least 55 % by weight of an isomer mixture of tertiary carboxylic acids having 9 carbon atoms, said carboxylic acids being so difficult to be esterified that the reaction product obtained by reacting said carboxylic acids with methanol in an amount 4 times the weight of the carboxylic acids in the presence of zinc acetate as a catalyst in an amount of 5.4 % by weight of the carboxylic acids at a reaction temperature of 200°C and a reaction time of 5 hours has according to gas chromatography a conversion rate of the starting carboxylic acids to the carboxylic acid esters of 40 % or less; and said carboxylic acid mixture includes sulfur of 0.020% by weight or less based on the total weight of the produced mixture said process comprises reacting a diisobutylene, carbon monoxide and water in the presence of a catalyst consisting of sulfuric acid and phosphoric acid under a pressure of 2-39 bar (2-40 kg/cm²), wherein the concentration of said sulfuric acid in the catalyst is 30-90 % by weight and the concentration of said phosphoric acid in the catalyst is 5-70 % by weight so that an acid strength by Hammett's acidity function Ho of the catalyst is within the range of from -9.2 to -6

3. The process according to claim 1 or 2, wherein said catalyst additionally contains a metal compound.

4. The process according to claim 3, wherein said metal compound is a metal oxide.

5. The process according to claim 3, wherein said metal compound is cuprous oxide.

6. The process according to claim 3, wherein the amount of said metal compound is 0.2-2 % by weight of the mixture of sulfuric acid and phosphoric acid.

## Patentansprüche

1. Verfahren zur Herstellung einer Carbonsäuremischung, welche mindestens 55 Gew.-% einer Isomerenmischung tertiärer Carbonsäuren mit 13 Kohlenstoffatomen enthält, wobei die Carbonsäuren so schwierig zu verestern sind, daß das durch Umsetzen der Carbonsäuren mit Methanol in einer Menge von dem 4-fachen des Gewichts der Carbonsäuren in Gegenwart von Zinkacetat als Katalysator in einer Menge von 5,4 Gew.-% der Carbonsäuren bei einer Umsetzungstemperatur von 200°C und einer Umsetzungszeit von 5 Stunden erhaltene Reaktionsprodukt eine mittels Gaschromatographie bestimmte Umwandlungsrate der Ausgangscarbonsäuren zu den Carbonsäureestern von 40 % oder weniger aufweist, wobei das Verfahren das Umsetzen von einem Triisobutylen, Kohlenmonoxid und Wasser in Gegenwart eines aus Schwefelsäure und Phosphorsäure bestehenden Katalysators unter einem Druck von 2-39 bar (2-40 kg/cm²) umfaßt, wobei die Konzentration der Schwefelsäure in dem Katalysator 30-90 Gew.-% und die Konzentration der Phosphorsäure in dem Katalysator 5-70 Gew.-% betragen, so daß die durch die Hammett'sche Aziditätsfunktion Ho bestimmte Säurestärke des Katalysators innerhalb des Bereichs von -9,2 bis -6 liegt.

2. Verfahren zur Herstellung einer Carbonsäuremischung, welche mindestens 55 Gew.-% einer Isomerenmischung tertiärer Carbonsäuren mit 9 Kohlenstoffatomen enthält, wobei die Carbonsäuren so schwierig zu verestern sind, daß das durch Umsetzen der Carbonsäuren mit Methanol in einer Menge von dem 4-fachen des Gewichts der Carbonsäuren in Gegenwart von Zinkacetat als Katalysator in einer Menge von 5.4 Gew.-% der Carbonsäuren bei einer Umsetzungstemperatur von 200°C und einer Umsetzungszeit von 5 Stunden erhaltene Reaktionsprodukt eine mittels Gaschromatographie bestimmte Umwandlungsrate der Ausgangscarbonsäuren zu den Carbonsäureestern von 40 % oder weniger aufweist; und wobei die Carbonsäuremischung Schwefel in einer Menge von 0.020 Gew.-% oder weniger, bezogen auf das Gesamtgewicht der hergestellten Mischung, beinhaltet; wobei das Verfahren die Umsetzung von einem Diisobutylen. Kohlenmonoxid und Wasser in Gegenwart eines aus Schwefelsäure und Phosphorsäure bestehenden Katalysators unter einem Druck von 2-39 bar (2-40 kg/cm²) umfaßt, wobei die Konzentration der Schwefelsäure in dem Katalysator 30-90 Gew.-% und die Konzentration der Phosphorsäure in dem Katalysator 5-70 Gew.-% betragen, so daß die durch die Hammett'sche Aziditätsfunktion Ho bestimmte Säurestärke des Katalysators innerhalb des Bereichs von -9,2 bis -6 liegt.

3. Verfahren nach Anspruch 1 oder 2, wobei der Katalysator weiterhin eine Metallverbindung enthält.

4. Verfahren nach Anspruch 3, wobei die Metallverbindung ein Metalloxid ist.

5. Verfahren nach Anspruch 3, wobei die Metallverbindung Kupfer(I)-oxid ist.

6. Verfahren nach Anspruch 3, wobei der Gehalt der Metallverbindung 0,2-2 Gew.-% der Mischung aus Schwefelsäure und Phosphorsäure beträgt.

## Revendications

1. Procédé de préparation d'un mélange d'acides carboxyliques qui contient au moins 55% en poids d'un mélange d'isomères d'acides carboxyliques tertiaires comportant 13 atomes de carbone, ces acides carboxyliques étant si difficiles à estérifier que le produit de réaction obtenu par réaction desdits acides carboxyliques avec du méthanol, en une quantité égale à 4 fois le poids des acides carboxyliques, en présence d'acétate de zinc comme catalyseur, en une quantité représentant 5,4% du poids des acides carboxyliques, à une température réactionnelle de 200°C et avec une durée de réaction de 5 heures, possède, selon une chromatographie en phase gazeuse, un taux de conversion des acides carboxyliques de départ en esters d'acides carboxyliques inférieur ou égal à 40%, ledit procédé comprenant la réaction d'un triisobutylène, de monoxyde de carbone et d'eau, en présence d'un catalyseur constitué d'acide sulfurique et d'acide phosphorique, sous une pression de 2-39 bars (2-40 kg/cm²), dans lequel la concentration dudit acide sulfurique dans le catalyseur est de 30-90% en poids et la concentration dudit acide phosphorique dans le catalyseur est de 5-70% en poids, de sorte que la force acide telle que définie par la fonction d'acidité de Hammett Ho du catalyseur soit dans la gamme de - 9,2 à -6.

2. Procédé de préparation d'un mélange d'acides carboxyliques qui contient au moins 55% en poids d'un mélange d'isomères d'acides carboxyliques tertiaires comportant 9 atomes de carbone, ces acides carboxyliques étant si difficiles à estérifier que le produit de réaction obtenu par réaction desdits acides carboxyliques avec du méthanol, en une quantité égale à 4 fois le poids des acides carboxyliques, en présence d'acétate de zinc comme catalyseur, en une quantité représentant 5,4% du poids des acides carboxyliques, à une température réactionnelle de 200°C et avec une durée de réaction de 5 heures, possède, selon une chromatographie en phase gazeuse, un taux de conversion des acides carboxyliques de départ en esters d'acides carboxyliques inférieur ou égal à 40% et ledit mélange d'acides carboxyliques comprenant 0,020% en poids ou moins de soufre par rapport au poids total du mélange produit, ledit procédé comprenant la réaction d'un diisobutylène, de monoxyde de carbone et d'eau, en présence d'un catalyseur constitué d'acide sulfurique et d'acide phosphorique, sous une pression de 2-39 bars (2-40 kg/cm²), dans lequel la concentration dudit acide sulfurique dans le catalyseur est de 30-90% en poids et la concentration dudit acide phosphorique dans le catalyseur est de 5-70% en poids, de sorte que la force acide telle que définie par la fonction d'acidité de Hammett Ho du catalyseur soit dans la gamme de - 9,2 à -6.

3. Procédé selon la revendication 1 ou 2, dans lequel ledit catalyseur contient aussi un composé métallique.

4. Procédé selon la revendication 3, dans lequel ledit composé métallique est un oxyde métallique.

5. Procédé selon la revendication 3, dans lequel ledit composé métallique est l'oxyde cuivreux.

6. Procédé selon la revendication 3, dans lequel la proportion dudit composé métallique est de 0,2-2% en poids du mélange d'acide sulfurique et d'acide phosphorique.
